# EUROPEAN PATENT APPLICATION

(11) **EP 0 604 697 A1**
(43) Date of publication of application: **06.07.1994**
(21) Application number: 93103935.8
(22) Date of filing: 11.03.1993
(51) Int. Cl.: A61B 17/60

(54) **Apparatus for external skeletal fixation**

(30) Priority: 16.12.1992 YU 1073/92
(71) Applicant: Mitkovic, Milorad, Dr., YU-18000 Nis (YU)
(72) Inventor: Mitkovic, Milorad, Dr., YU-18000 Nis (YU)

(57) **Abstract**

This invention belongs to the field of the medical science - surgery and related to the device for external skeletal fixation, for reduction of the fractures and treatment of different skeletal deformations (A 61 b 17/60).

By this invention it is solved the problem of the application of the pins (25), parallel or with different angle between each other, without any kind of the guidance, giving to them three dimensional freedom of the movement and giving nearly equal biomechanical conditions in any plane. Device is high mobile. It is necessary to introduce at list two pins (25) in every fragment of the bone (28).

Essence of the invention is in the construction of the complex of the carrier (1) with clamp (2). The number of such complexes corresponds to the number of the pins - one complex for each pin. By the construction it has been realized sliding and rotatory movement of the clamp (2) round and along the body (4) of the carrier (1), and same is possible with carrier (1) which are by its holes (6) pulled on to the bar (3). In other hand by the canals (24) clamps (2) are pulled on to the pins (25). Adjustment of all components each other makes possible three dimensional movements of the pins (25), and after adjustment all components are fixed by the tightening of the nuts (11, 26). So bone fragments are fixed in desirable position which were the goal of this invention.

## Description

The invention relates to a fixation of bones fractures, alignment of the bone fragment and treatment of different skeletal deformities and morphologic abnormalities by simplifying means.

According to international classification of the patents (MKP4) this invention is determined by the basic symbol of the classification A 61 B 17/60. By this symbol, devices and instruments for external fixation for application in surgically methods are marked.

External skeletal fixators make it possible to fix bone fragments by screwing the pins or wires introducing into the bone fragments, out of fracture region, avoiding so introducing of foreign materials to the fracture site.

A lot of types of external fixators exist. All of them make it possible to fix bone fragments after achieving reduction of dislocations by opening of the fracture region. Only few of them make possible to do not only fixation of the bone fragment but reduction of the fracture (Ortofix, Hoffmann's, Scherer's, Ilizarov's etc.). These last systems posses limited freedom for pin application. It means:
1. Special guidance have to be used for pin application or apparatus itself have to be used as a guidance. So, time for application is prolonged and errors surgeon did are not rare.
2. Pins in the most known external fixators like Ortofix and Hoffmann-Vidal have to be parallel which means that apparatus is 5 - 20 times less stable in the plane on the right angle to the pins then in the plane of the pins. So there is not optimal biomechanical conditions for bone healing.

Apparatuses for fixation of small bones have two lackes: they are to complicated or low mobile.

The main goal of this invention is in the solving of the problem of the application of the pins without any kind of guidance, whereby device have to by high adjustable and to be with equal stability in any plane. Device also have to be simple for handling, even for less skill surgeons.

Essence of the invention is in achieving three dimensional freedom of the moving of each pin. Frame of the apparatus can be always applied to the introduced pins independently of its positions - even if they are applied in random from parallel to 120 degrees. It is possible thanks to specially designed clamps, turningly point fixed to its carriers, whereby, through the holes of the carriers bar or tube is fixed. This bar or tube holds all elements in one construction. After skin incision, two to six thread pins are screwing. Pins can be parallel or convergently orientated having reference to any of three planes (frontal, sagittal and horizontal). The most optimal biomechanical conditions are if pins are convergent from 60 to 90 degrees in horizontal plane. After attaching of the frame that is bar with carrier of the clamps and clamps, one high mobile external fixator is forming. After reduction of the fracture is achieved, assistant definitively fixes all components.

This external fixation device has many advantages as:
- Apparatus is high mobile and has equal stability in any plane,
- During the application of the pins it is not necessary to use any kind of guidance,
- Each pin can be moved three dimensionally,
- The frame can be easily attached to the pins even if the pins were randomly introduced in bone,
- Possibility of the infection is minimized,
- Application of this external fixator can be performed for 10 minutes,
- Addition reductions are possible and
- handing of this external fixator is simple, thanks to small number of components.

The advantages offered by the invention are mainly that telescopic clamp gives independent 3 dimensional freedom for each pin application; frame of the external fixator can be attached after pins have been arbitrarily introduced between 0 and 120 degrees. External fixator can be applied for 10 minutes which makes possible fast and safe solving of multiple bone fractures without opening of the fracture region. Correction of reduction is also extremely simplifies by the use of double joint. Low number of components also simplified the using of this system.

Several ways of carrying out the invention is described in details below, with reference to drawings which illustrate several specific embodiments, in which:

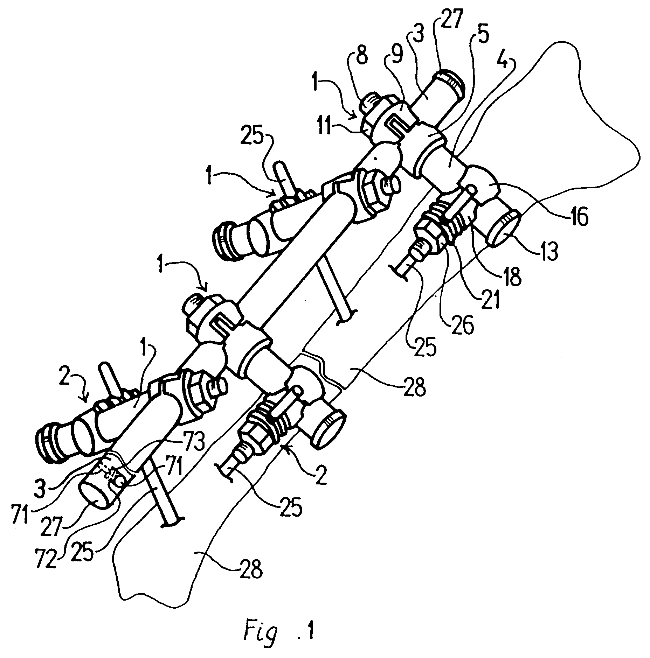

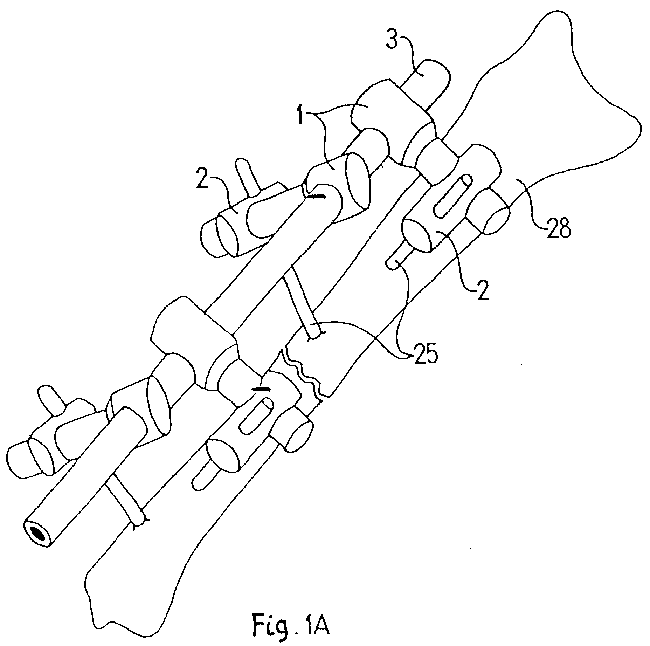

Figure 1A is a perspective view of the external fixator in accordance with the invention.
Figure 1 is a perspective view of the external fixator in accordance with the invention, with all elements and bone.
Figure 2 is a perspective view of the carrier of the telescopic clamp.
Figure 3 is a perspective view of the clam.
Figure 4 is a cross section drawings of double joint with wedge locking mechanism.
Figure 5 is a cross section drawings of double joint with wedge locking mechanism along the V-V line from figure 4.
Figure 6 shows vertical view and partially cross section of the cylindric clamp.
Figure 7 shows horizontal view of the cylindric clamp from figure 6.
Figure 8 shows profile projection of the cylindric clamp from figure 6.
Figure 9 is perspective view of the half ring carrier of the clamp in connecting position with one bar and one cylindric clamp.
Figure 10 is perspective view of the half ring-clamp connecting screw.
Figure 11 shows a complex view of the invention according to variant 1, led out with ball like joint clamps for fixing of two pins, in perspective view.
Figure 12 shows a one ball like clamp in horizontal projection from figure 11.
Figure 13 is a drawing of the hemicortical pin.

Complex of the device for external fixation according to the invention is shown in the figure 1A and 1, whereby device is in the position, for example, for reduction and fixation of the fracture. Device has 3 basic components: carrier of the clamp 1, clamp 2 and bar 3.

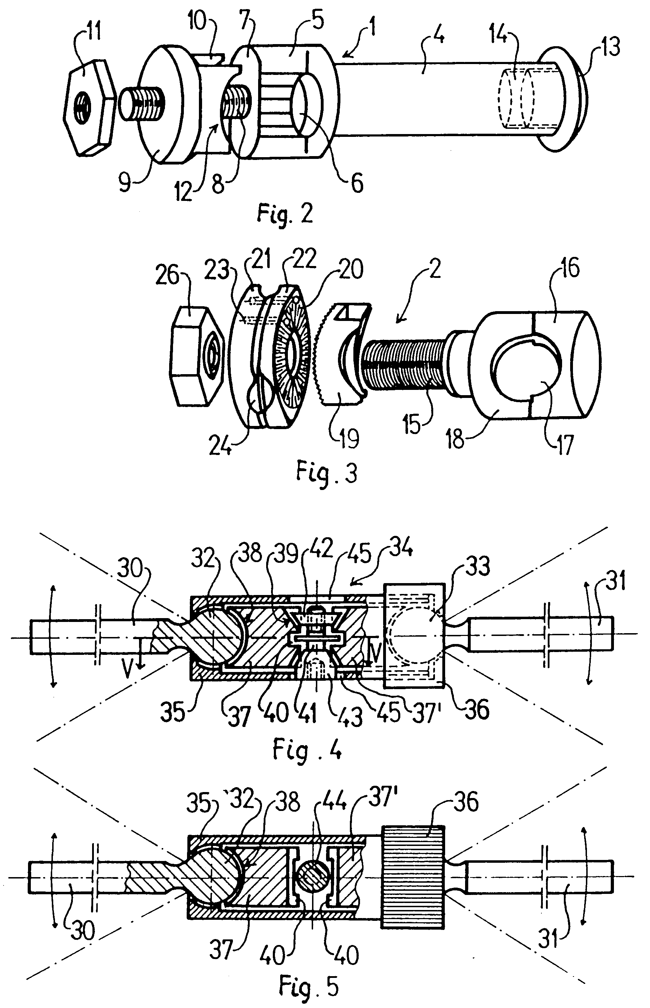

Carrier of the clamp 1 (fig. 2) has cylindric body 4 with cylindric like enlarged head on one end 5. In the head cylindric hole were made 6 whereby axis of this hole 6 is in position of right angle to to long axis of the body 4. Body 4 is carrier of the complex clamp 2, while through hole 6 bar 3 is introduced. Cylindric like head 5 is on the both lateral side symmetrically cut, from its outer end to approximately middle of the hole 6, forming so saddle 7 to which from the out, coaxially to body 4, is thread extension 8. Above the thread extension 8 to the saddle 7 is pulled on cylindric rider 9 with its section 10 which clasps saddle 7, whereby lateral ends 12 of the rider 9 are half circularly designed so that all together with saddle 7, partially pass to hole 6 and serves for fastening of the introduced bar 3. Fastening of the bar 3 is performed by the nut 11 which, by screwing on the thread extension 8, push rider 7 which end 12 press bar 3. In order to stop slipping out of the carrier of the clamp 1 from the bar 3 and clamp from the carrier of the clamp 4, identical screws 27 and 13 with oval head which has bigger diameter than diameter of 3 and 4 are, is screwed. Bar 3 and body 4 has same diameter. Screw 27 and 13 is screwed in threaded hole ends of the 3 and 4 (see fig. 2).

Clamp 2 shown to the figure 3 has special screw 15 with supporting head 16 which is identical with supporting head 5 above described carrier 1 of the clamp. Through supporting head 16 there is one cylindric hole 17, whereby supporting head 16 on the both lateral side is symmetrically cut, from its outer end to approximately middle of the hole 17, forming so saddle 18. To the saddle 18 it is pulled on cylindric rider 19 which is identical to the rider 9 of the carrier 1 with its section 10 which clasps saddle 18. Only one difference between the riders 9 and 19 is, that rider 19 on the round ring like outer surface has teeth (it is not visible on the figure. In the complex with supporting head 16 rider 19 presses in the hole 17 to the body 4 of the carrier 1. After rider 19 is pulled on, two round ring like plates 21 and 22, each same diameter, are pulled on and they are shaky connected with two axle which are riveted.

Surfaces of the opposite sites of the plates 21 and 22 there are gutters one opposite other whereby forms canal 24 for pulling on of the clamp 2 to the pin 25. The fixing of the clamp 2 to the pin 25 is performed by the screwing of the nut 26 on the screw 15 (see fig.1) pressing so the plate 21 to plate 22, and at same time plate 22 press by its surface to the rider 19, which by moving into the hole 18, make fixed entirely clamp 2 to the body 4 of the carrier 1.

The surface of the plate 22 and surface of the rider 19 which are opposite and which are in the contact, on the round ring like outer surface have identical teeth 20 (teeth on the rider are not visible), so that in fixed complex it not possible slipping between the plate 22 and rider 19.Pin in the gutter 24 can be fastened in any circular position.

The bar 3 serves for connecting of the all carriers 1 of the clamps 2 and it is circular cross section shape. On the ends of the bar or tube 3 in its threaded holes, in order to stop slipping out of the carrier of the clamp 1 from the bar 3 screws 27 with oval heads with bigger diameter than diameter of 3 is, is screwed. The application technique of this external fixator, according to this invention, is from above description clear, specially to the orthopaedic surgeons. In the figure 1 this external fixator was applied to the one long bone 28.

After ordinary preparing of the operating field and working out of the eventual wound, approximate reduction of the fracture is performed (by the open or closed method). On the skin, 4 incision are made for pins 25. After that four holes are made by the drill without any kind of the guidances. In each hole one pin is screwed 25. Pins can be placed in any position having reference to each other or having reference to the long axis of the bone 28. The most desirable biomechanical conditions are if convergence is between 60 and 90 degrees. Before operation, frame is preparing so that screw 27 is unscrewed from the bar 3 and four carriers of the clamps 1 are pulled on. Before that nut 11 is unscrewed so that hole 6 becomes bigger. After that screw 27 is screwed. Before pulling on of the carrier of the clamp 1, one clamp 2 is pulled on to to the bodies 4 of the each carrier of the clamp 1 by unscrewing of the screw 13. This screw is returned after that to its place 14.

Screws 13 and 27 serves for stopping components 1 to slip out of from bar 3 and 2 from carrier of the clamp 4. Instead that screw, on the each end can be inserted little ball 71 visible to the figure 1. This ball is pressed by the spring 72 in canal 73. Canal is narrower in the end where the ball 71 is, so that ball 71 can not slip out from the canal while the part of the rest of the canal is with smaller diameter 74.

Finally, to the applied pins frame is attached, so that clamp 2 are pulled on to the pins 25 through the holes formed from the gutters 24 of the plates 21 and 22.

After frame application, surgeon makes reduction of the fracture while surgeon assistant tightens all nuts 11 and 26.

If additional reductions are needed they are possible by the loosening of the clamps 1 and carriers of the camps 2 by which only one fragment fixed is (unscrewing of the nuts 11 and 26). Removing of the frame during the healing process and its returning in order to checking of firmness of the callus is also possible.

On the figure 4 and 5 it is shown according to the invention another variant of the bar 3 which is in here marked as 29 and consists of two parts 30 and 31, circular cross section, whereby each of part 30 and 31 has one ball like end 32, 33. Balls 32 and 33 as ends of 30 and 31 are parts of the double joint 34 in which they are. Parts 30 and 31 can be identical or with different sizes and designs. Double joint 34 is placed in the cylindric homesite 35 which inner diameter is a little bigger than diameter of the ball 32 or 33. One end of the homesite 35 has ring like shape which diameter is little narrower than diameter of the ball 32 or 33 while other end of the homesite is closed by the cylindric nut 36 from outside, whereby nut 36 has narrower hole same diameter as diameter of the hole on the ring like end of the homesite 35.

Folding of the double joint 34 is very simple. First nut 36 is taken off from homesite 35. Then through homesite is puller through tube like or bar like part 30 until ball 32 is placed in the concave end of the homesite 35, whereby tube 30 is then completely outside. Then in the homesite 35 is thrown into speciall pressing element 37 which with its concave shape 38 is in contact with ball 32, while the opposite end of the pressing element 37 has shape with two identical oblique and opposite orientated surfaces 39 cut with one canal 40. On the pressing element 37 it is placed specially designed screw 41 to which it is screwed one plate like wedge 42 which oblique surfaces are in contact with oblique surfaces 39, while above the head 43 of the screw 41 there is integrally ring like plate 44 which go to canal 40 in order to limit the movements of the screw 41. After that over the screw, another concave pressing element 37', identical to the previously described 37, but opposite orientated. Through cylindric nut 36 is pulled through another 31 which ball like end 33 is in contact with spheric inner end of the cylindric nut 36, which is after that screwed to the homesite 35. In order that screw 41 can be screwed, in its head there is hexagonal hole for corresponding imbus key, which is introduced through elliptic hole 45 of the homesite 34 in direction of the long axis of the screw 41.

By the screwing of the screw 41 wedge 42 go in direction to the head pressing with its oblique surfaces to pressing elements 37 and 37' producing so squeezing and impacting of the balls 32, 33 in the homesite 35 in the desirable position. By the unscrewing of the screw 41 wedge 42 go to opposite direction making free the pressing elements 37 an 37' and so bars 30 and 31. Construction of the double joint makes possible that bars 30 and 31 can take any position in the space limited by the cone which angle is 60 degrees.

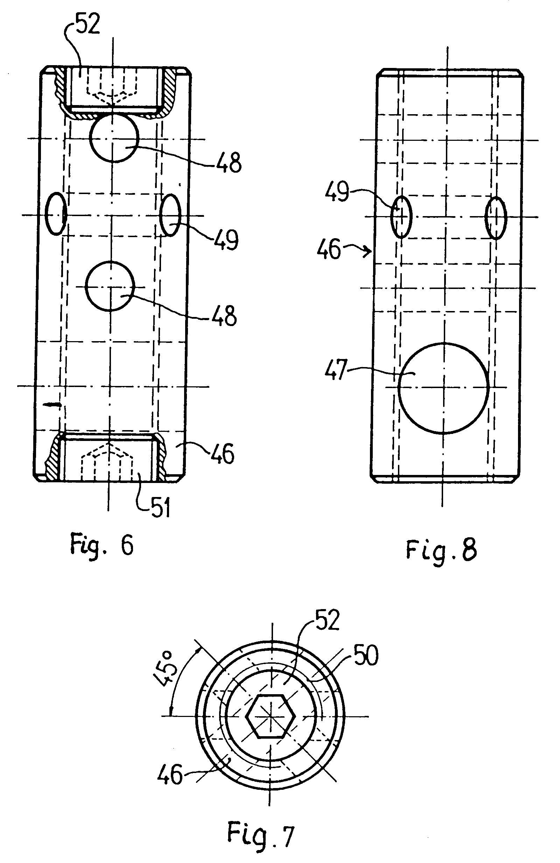

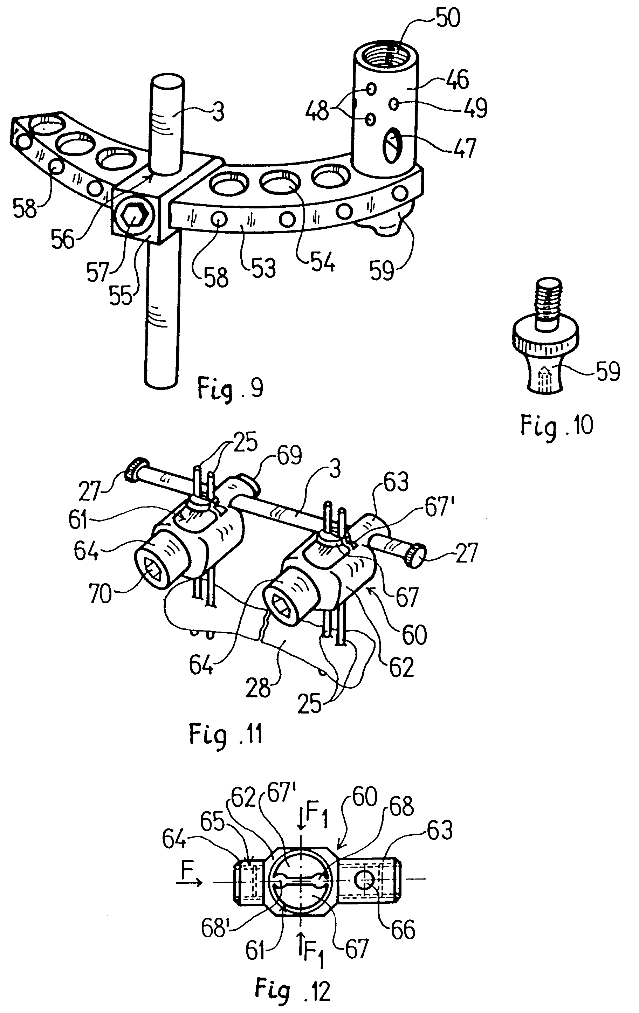

On the bars 30 and 31 it possible to pull on previously described carrier of the clamps 1 or clamps 2 or cylindric clamps 46 (figure 8) or ball like clamps 60 (figure 11).

On the figures 6, 7 and 8 it is shown according to the invention cylindric clamp 46. On its end this clamp has bigger hole 47 for bar 3 or 30, 31 or 29, whereby axis of the hole 47 is at the right angle to the long axis of the clamp 46. Also in this clamp there are four holes more. Two are parallel 48 and another two holes 49 are convergently orientated and axis of each has 45 degrees angle having reference to the axis of the holes 48. Axis of the holes 48 are at the right angle to the long axis of the clamp 46 and to the right angle to axis of the hole 47. All inner surface of the clamp 46 has thread 50 for screw 51 for fixation of the bar 3 or 29 and pin 25 which can be pulled through any of the holes 48 or 49.

In the figure 9 it is shown according to the invention half ring like carrier 53 for the clamp 46. This half ring 53 has holes 54 and flat surfaces 58. Each flat surface 58 correspond to one hole 54 so that center of the 54 is in the same axis as center of the 58 and this axis represent one radius of the half ring 53. To half ring it is pulled on one tightener 55 which has coaxial holes 56 which are identical to holes 54. This tightener 55 can be pulled on to any hole 54. Through the hole 56 of the tightener 55 and through the hole 54 of the half ring it is pulled through the bar or tube 3 or 30, 31. Bar 3, 30 or 31 can be fixed in any position by the screwing of the screw 57 which press with its flat end surface to the flat surface 58 whereby is produced squeezing and impacting of the bar - half ring connection.

Thorough any of the holes 54 it can be pulled through special screw 59 fig. 10 in order to be screwed into the threaded inner surface of the clamp 46. So it is achieved fixation of three elements: half ring 53, clamp 46 and screw 59.

On the figures 11 and 12 it is shown according to the invention a ball joint clamp for two pins 60. This clamp comprises: two half balls 67, 67' (one ball, cut to two half balls), capsule 62, cylindric extension for screw 64, which is for fixing of the ball 70, cylindric extension 63 with the hole 66 for the bar, screw for fixing of the bar 69 and two canals 68, 68' (formed from two gutters on each half balls for two pins 25. Screw 70 has concave shape surface in the end which is in contact with one or two half balls. Inner surfaces of the capsule 62 are concave shaped for contact with two half balls 67, 67'. Between the two half balls there is one spring which pushes two half balls in opposite directions in order to stop dropping out of the half balls 67, 67' from the capsule 62. Screws are without heads and imbus key is corresponding.

In accordance with the invention screwing of the 70 makes pressure to half balls 67, 67' producing so fixing of the half balls, the capsule 62, screw 70 and two pins 25. Pressure to the pins is produced by the screw 70 itself and by the making narrower of the capsule 62 because of its special shape. In the fig. 12 it is shown that pressing force F produced by the screwing of the 70, produces the two bilateral side forces F1 so that ball is pressed from four sides.

Folding of the clamp 60 is simple. Two half balls 67, 67' are joined first with spring between them (it is not shown) and then they are insertedthe capsule 62. Than screw 70 is screwed without tightening, bar 3 is pulled through hole 66 and each clamp 60 is pulled on to the par of the pins 25, before that, introduced into the bone 28 by the use of special guidance (is not shown). After reduction of the fracture all screws 70 and 69 are tightened.

Combinations of clamps 1, 2, 46, 60, bar or tube 3, double joint 34 with bar or tube like extensions 29, half ring 53, tightener 55 and screw 59 are possible in order to built one external fixation construction or to fold independent external fixators.

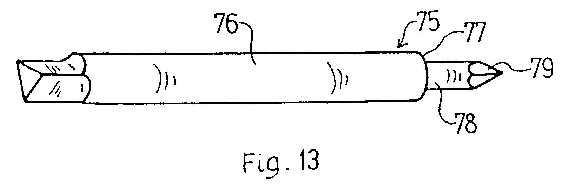

In figure 13 it is shown the pin which diameter is normal in almost all its body except on its end which go to the bone. Before the top of the pin its diameter rapidly become narrower 77 so that its diameter is smaller along the short distance 78 and it endes by the sharp top 79. Into the bone is introduced only this thinner part 78 avoiding so pin to pass through medullary canal of the bone and avoiding so to damage of the medullary bone vassals.

## Claims

1. Device for external fixation of the bones, which is attached to at list two pins (25) in each bone fragment (28), **pointed by that**, that comprises at list four identical complexes of the carriers (1) with clamps (2), whereby clamp (2), by its cylindric hole (17) pulled on to it, can to slide and to rotate along the cylindrical body (4) of the carrier of the clamp (1) to which it can be attached, that are complexes of the carriers (1) with clamps (2), can to slide and to rotate, by cylindric hole (6) on the carrier (1) pulled on to the bar (3) to which complexes can be fastened, that on the each applied pin (25) by possibility of sliding and rotating pulled on clamps (2) can be fixed by the nut (26) simultaneously to pin (25) and to the body (4) of the carrier (1) and that pins (25) through bone fragments (28) are applied without using of any kind of guidance, the most optimal in the convergent orientation with angle between them from 60 - 90 degrees.

2. Device according the claim 1**, pointed by that**, that on the cylindric body (4) of the carrier (1) continues carrying head (5) with cylindric hole (6) which is bilateral symmetrically cut forming so saddle (7) to which coaxially with the body (4) continues thread part (8), that on the thread part (8) to saddle (7) pulled on cylindric rider (9) by its section (10), whereby bilateral ends (12) of the rider (9) are half circular shaped in order to clasp and to fast the bar (3) by tightening of the nut (11) on the part (8) and that on the free end of the body (4) is screwed screw (13) with lens like head which diameter is bigger than diameter of the body (4) or balls (71) thrown into the hole (73) with spring (72) whereby spring (72) presses to ball (71) which can not slip out because of smaller diameter of the outer end of the hole (73).

3. Device according to the claim 1, **pointed by that**, that clamp (2) has special screw (15) with carrying head (16) which has cylindric hole (17), whereby head (16) is bilaterally symmetrically cut forming saddle (18) to which is pulled on rider (19) with teeth (20), that on the screw (15) to the rider (19) pulled on two circular ring like plates (21, 22), shaky connected with two axle (23), which in the complex make canal (24) for the pulling on of the clamp (2) to the pin (23) to which it is fastened by the tightening of the nut (26) to the screw (15).

4. Device according to claim 1 and second manner of the performance, **point ed by that**, that bar (29) has two parts (30, 31) which can to move and to rotate, with ball like ends (32, 33) into the spherical ends of the cylindric homesite (34) including its nut for closing (36) and that two identical pressing elements (37, 37') inserted in the homesite (35), have concave surfaces (38) on its outer ends, while frontal parts of their central ends (37, 37') have two opposite orientated and bordered surfaces (39) cut by the rectangular canal in the middle (40), and between the pressing elements (37, 37') there is special screw (41) with screwed plate like wedge (42) which oblique surfaces are in contact with correspond oblique surfaces (39), and by the circular limited plate (44) which is between the head (43) of the screw (41) and wedge (42) whereby plate (44) is into the rectangular canal (40), whereby bilateral on the cylindric homesite (35) are elliptic holes (45) for approach to the imbus hole of the head of the screw (43) for screwing and unscrewing of the screw (41).

5. Device according to the claims 1 and 3 and according to second manner of the performance, **pointed by that**, that cylindric clamp (46) has, on one end, hole (47) for the bar (3) or for lever (30, 31) of the bar (29), two parallel holes (48) and two holes, with the convergency of 45 degrees (49) having reference to (48), whereby holes (48, 49) serves for pulling on of the cylindric clamps (46) to the pins (25) and that to the bought ends of the cylindric clamp (46) in the inner threads (50) screwed screws (51, 52) for tightening of the bar (3, 29) and the pins (25).

6. Device according to the claims 1 and 2 and according to second manner of the performance, **pointed by that**, that on the ring like segmental carrier (53) there are several identical, with equal distance between each other and with parallel circular holes (54), whereby prismatic tightener (55) through its section is pulled on to one of the holes (54) for pulling on to the bar (3), that on the front of the tightener (55) is threaded hole for screw (57) for tightening of the bar (3) in the holes (54, 56) and that to one of the hole (54), coaxially with it, can be placed cylindric clamp (46) which by the special screw (59) can be fastened to the segmental carrier - half ring (53).

7. Device for external skeletal fixation according to the variant I, **pointed by that**, that carrier (60) of the clamp (61) performed as capsule (62) with concave inner surfaces, whereby capsule (62) has bilateral shorter and longer cylindric extensions (63, 64) with inner threads (65), that on the longer extension is performed side hole (66) for bar (3), whereby in the homesite of the capsule (62) inserted clamp (61) from two identical half balls (67, 67') with two parallel gutter at each, forming in complex approximately cylindric canals (68, 68') for pulling on of the clamp (61) to the pare of the pins (25) and that into the longer extension (63) is inserted screw (69) for tightening of the bar (3), while into the shorter extension (64) is inserted screw (70) for tightening of the pins (25) through inner thread (65) of the carrier (60).

8. Pin for external skeletal fixation, **pointed by that**, that diameter of its body (76) is wider, whereby before its one end rapidly become narrower (77) so that its diameter further is smaller along the short distance (78) finishing with sharp top (79).
